# EUROPÄISCHE PATENTSCHRIFT

(11) **EP 1 098 599 B1**
(45) Veröffentlichungstag und Bekanntmachung des Hinweises auf die Patenterteilung: **01.09.2004**
(21) Anmeldenummer: 99936286.6
(22) Anmeldetag: 18.05.1999
(51) Int. Cl.: A61B 18/20

(54) **LASERINSTRUMENT**
LASER INSTRUMENT
INSTRUMENT A LASER

(30) Priorität: 18.05.1998 DE 19821986
(43) Veröffentlichungstag der Anmeldung: 16.05.2001
(73) Patentinhaber: Dornier MedTech Holding International GmbH, 82234 Wessling (DE)
(72) Erfinder: STILLER, Hans-Peter, D-85221 Dachau (DE)
(74) Vertreter: Grünecker, Kinkeldey, Stockmair & Schwanhäusser Anwaltssozietät
(86) Internationale Anmeldenummer: PCT/DE1999/001507
(87) Internationale Veröffentlichungsnummer: WO 1999/059483

(56) Entgegenhaltungen:
- EP-A- 0 325 836
- EP-A- 0 404 968
- EP-A- 0 514 258
- US-A- 5 416 878
- US-A- 5 693 043

## Beschreibung

Die Erfindung bezieht sich auf ein Laserinstrument nach dem Oberbegriff des Patentanspruchs 1.

Laserinstrumente dieser Art werden zumeist in Verbindung mit einem Endoskop für nicht- oder minimalinvasive Eingriffe zur Kontaktvaporisation von biologischem Gewebe vor allem in englumigen Hohlorganen, etwa zur transurethralen Geweberesektion der Prostata bei benigner Prostatahyperplasie eingesetzt. Bei einem derartigen, aus der EP 0 514 258 A1 bekannten Laserinstrument ist die distalseitig am Laserlichtleiter befestigte Applikationskappe gemeinsam mit dem Lichtleiterende abgebogen, so daß die Laserstrahlung in Form eines bezüglich der Instrumentenachse schräg geneigten Strahlenkegels an der Applikationsspitze austritt. Dabei besteht die Gefahr, daß die Applikationskappe während der Behandlung unkontrolliert in Richtung der Applikationsspitze, also seitlich zur Verschieberichtung, abdriftet, so daß eine gezielte, patientenschonende Gewebeabtragung in einem eng umgrenzten Behandlungsgebiet stark erschwert oder gar unmöglich ist.

Ferner ist aus der EP 0 433 464 B1 ein laserchirurgischer Applikator mit einer abgebogenen Applikationsspitze bekannt, welche örtlich mit strahlungsabsorbierenden und lichtstreuenden Partikeln beschichtet ist, um so in einzelnen Flächenabschnitten des Applikators unterschiedliche Behandlungswirkungen, nämlich zum einen einen mehr koagulierenden und zum anderen einen mehr schneidenden Strahlungseffekt zu erzielen, wobei die hochgebogene Applikationsspitze in den biegeäußeren Flächenbereichen hauptsächlich streulichtbeleuchtet ist, so daß sich dort eine schwache Vaporisationswirkung ergibt und dieser Applikator somit nur sehr begrenzt für eine effektive Gewebeablation einsetzbar ist.

Aufgabe der Erfindung ist es, das Laserinstrument der eingangs genannten Art so auszubilden, daß eine effektive Gewebevaporisation sowohl in prograder als auch in lateraler Richtung bezüglich der Instrumentenachse und zugleich eine Selbststabilisierung der Applikationskappe bei der Gewebeabtragung erzielt werden.

Diese Aufgabe wird erfindungsgemäß durch das im Patentanspruch 1 gekennzeichnete Laserinstrument gelöst.

Bei dem erfindungsgemäßen Laserinstrument wird aufgrund der besonderen geometrischen Gestaltung der Applikationskappe die Austrittsfläche des Laserlichtbündels signifikant, nämlich über den gesamten biegeäußeren Flächenbereich zumindest bis zur äußersten distalen Spitze, nicht aber über den radial äußersten Flächenpunkt des Biegeteils hinaus aufgeweitet und dadurch bei nur geringer Vergrößerung der Außenabmessungen der Applikationskappe eine äußerst effektive Gewebevaporisation erreicht, und zwar nicht nur seitlich, sondern auch in Richtung der Instrumentenachse, so daß zusätzlich zu einer raumgreifenden Gewebeabtragung auch eine Rekanalisation von stenösen Hohlorganen problemlos möglich ist, mit dem weiteren wesentlichen Aspekt, daß der biegeinnere Teil der abgebogenen Applikatorfläche aus dem Strahlengang des Laserlichtbündels ausgeblendet ist und durch die damit erzielte Verteilung von vaporisierenden und unbestrahlten Applikator-Teilflächen in Verbindung mit dem Gleiteffekt des Biegeteils eine Selbststabilisierung der Applikationskappe gewährleistet und die Gefahr einer unkontrollierten Gewebevaporisation oder eines Verklebens von Gewebe an der Applikatorspitze weitgehend abgebaut wird.

Eine besonders vorzugsbehaftete Ausgestaltung gemäß Anspruch 2 sieht vor, daß zumindest der Bereich des Biegeteils, aus dem der Strahl austritt aufgerauht ist. Hierdurch werden beim Bewegen des erfindungsgemäßen Laserinstruments geringe Teile das zu vaporisierenden Gewebes mechanisch abgeschabt und bleiben in der aufgerauhten Oberfläche des Biegeteils haften. Unter dem Einfluß der Laserstrahlung beginnen diese Gewebeteile zu karbonisieren und die absorbierte Laserstrahlung erzeugt direkt an der Oberfläche des Biegeteils eine Temperatur, welche das mit dem Biegeteil in Berührung kommende Gewebematerial schnell vaporisieren läßt.

In vorteilhafter Ausgestaltung der Erfindung ist der axiale Kappenteil gemäß Anspruch 3 proximalseitig als den Faserkern des Lichtleiters mit radialem Abstand umschließende Aufnahmehülse ausgebildet, was den Vorteil hat, daß der Lichtleiter in geschützter Lage in der Applikationskappe untergebracht ist und zugleich eine sichere Laserlichtführung im Lichtleiter bis zum distalen Lichtleiterende erhalten bleibt. Dabei ist der Lichtleiter gemäß Anspruch 4 vorzugsweise mit dem proximalen Ende der Aufnahmehülse verklebt und diese ist entsprechend der Hülsenlänge als thermische Isolationszone ausgebildet, wodurch zum einen eine hochfeste mechanische Verkoppelung zwischen Lichtleiter und Applikationskappe erreicht und zum anderen wärmeempfindliche Einzelteile, wie etwa die Klebestelle, am proximalen Applikatorende wirksam gegen eine Überhitzung geschützt werden.

Nach Anspruch 5 wird das Laserinstrument vorzugsweise in Verbindung mit einem Endoskop verwendet und dabei die Applikationskappe im Arbeitskanal des Endoskops verschieblich geführt, um so vom proximalen Lichtleiterende aus Druck auf das Gewebe ausüben zu können und eine zielgenaue Handhabung zu ermöglichen. In diesem Fall wird die Länge der Aufnahmehülse nicht nur unter dem Gesichtspunkt der thermischen Isolationszone, sondern nach Anspruch 6 zweckmäßigerweise auch so bemessen, daß die Applikationskappe mittels der Aufnahmehülse über den gesamten Verschiebeweg im Endoskop geführt ist.

Im Hinblick auf eine optisch hochwertige Verkoppelung von Lichtleiter und Applikationskappe ist das distale Lichtleiterende gemäß Anspruch 7 zweckmäßigerweise mit dem axialen Kappenteil verschmolzen, und um Reflexionen an der Lichtleiter-/Applikator-Grenzfläche zu minimieren, bestehen die Applikationskappe und der Faserkern des Lichtleiters nach Anspruch 8 zweckmäßigerweise aus Materialien mit identischem Brechungsindex, und zwar aus Gründen einer hohen thermischen Belastbarkeit nach Anspruch 9 vorzugsweise aus Quarzglas.

Aus Herstellungsgründen ist der Biegeteil nicht stetig gekrümmt, sondern verläuft nach Anspruch 10 zweckmäßigerweise abgewinkelt zum axialen Kappenteil, und zwar unter einem Biegewinkel zwischen 5° und 30°.

Aufgrund seiner exakt steuerbaren, patientenschonenden Vaporisationswirkung ist das erfindungsgemäße Laserinstrument vielseitig verwendbar, wird jedoch in besonders bevorzugter Weise zur transurethralen Prostatabehandlung, insbesondere bei benigner Prostatahyperplasie, eingesetzt.

Die Erfindung wird nunmehr anhand eines Ausführungsbeispiels in Verbindung mit den Zeichnungen näher erläutert. Es zeigen in stark schematisierter Darstellung:
- **Fig. 1**: eine teilweise geschnittene Seitenansicht eines erfindungsgemäßen Laserinstruments im Bereich des distalen Applikatorendes; und
- **Fig. 2**: einen Gesamtschnitt des Applikators und des Lichtleiters in Verbindung mit dem distalen Ende des Arbeitskanals eines Endoskops.

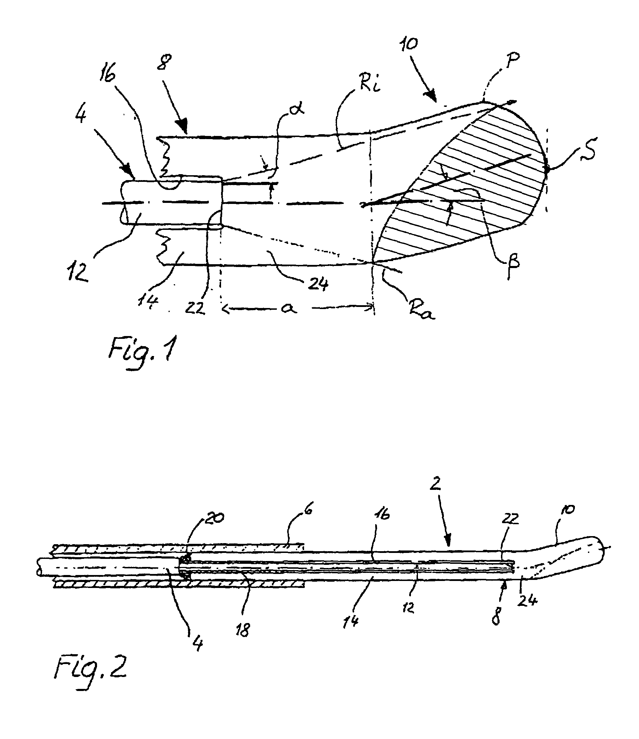

Das in den Fig. gezeigte Laserinstrument enthält als Hauptbestandteile eine Applikationskappe 2 und eine Lichtleitfaser 4, die gemäß Fig. 2 im Arbeitskanal 6 eines Endoskops untergebracht ist. Der proximalseitige Anschluß der Lichtleitfaser 4 an eine Laserlichtquelle ist ebenso wie die übrigen Teile des Endoskops einschließlich der Spülmittelkanäle und der visuellen Kontrollfunktion von üblicher Bauart und daher in den Fig. nicht näher dargestellt.

Die Applikationskappe 2 besteht aus einem zylindrischen, zur Lichtleitfaser 4 koaxialen Kappenteil 8 und einem von diesem abgewinkelten, ebenfalls zylindrischen, am distalseitigen Ende abgerundeten Biegeteil 10 und ist aus dem gleichen Material wie der Faserkern 12 der Lichtleitfaser 4, nämlich Quarzglas mit gleichem oder annähernd gleichem Brechungsindex gefertigt. Am proximalseitigen Ende bildet der axiale Kappenteil 8 eine Aufnahmehülse 14 für die Lichtleitfaser 4, wobei das Lumen 16 der Aufnahmehülse 14 im wesentlichen den gleichen Durchmesser wie die Umhüllung 18 der Lichtleitfaser 4 besitzt. Mittels einer Verklebung 20 zwischen Umhüllung 18 und Aufnahmehülse 14 ist die Lichtleitfaser 4 mechanisch fest und dichtend mit der Applikationskappe 2 verbunden, jedoch ist die Umhüllung 18 unmittelbar hinter der K lebestelle entfernt, so daß nur noch die reine Lichtleitfaser (Faserkern 12 und ggf. Fasermantel) unter Freilassung eines Ringspalts zum distalen Ende der Aufnahmehülse 14 verläuft, wo das Lichtleiterende 22 aus Gründen einer qualitativ hochwertigen, möglichst reflexionsarmen Laserlichteinkoppelung mit dem Kappenteil 8 verschmolzen ist.

Wie aus Fig. 1 am deutlichsten ersichtlich, ist der axiale Kappenteil 8 über die Verschmelzungsfläche mit dem distalen Lichtleiterende 22 hinaus verlängert, und die Länge a des verlängerten Kappenabschnitts 24 ist in Abhängigkeit von dem Divergenzwinkel α des Strahlenbündels im Kappenteil 8 so bemessen, daß der biegeäußere Randstrahl Ra des Strahlenbündels im Übergangsbereich zwischen axialem Kappenteil 8 und Biegeteil 10 austritt. Der biegeinnere Randstrahl Ri des Strahlenbündels in der Kappe 2 hingegen verbleibt im Kappenteil 8 und im Biegeteil 10 und tritt erst am abgerundeten Ende des Biegeteils 10 zwischen der äußersten distalen Spitze S und dem radial äußersten Flächenpunkt P des Biegeteils 10 aus. Zu diesem Zweck ist der Biegewinkel β des Biegeteils gleich dem oder größer als der Divergenzwinkel α, unter Berücksichtigung der Länge des Biegeteils 10 aber so klein gewählt, daß der gesamte, biegeäußere (in Fig. 1 schraffierte) Flächenbereich des Biegeteils 10 einschließlich der distalen Applikationsspitze S, nicht jedoch über den radial äussersten Flächenpunkt P hinaus vom Therapielicht ausgeleuchtet wird und die übrigen Flächenbereiche des Kappen- und des Biegeteils 8 und 10 vom Strahlengang des Laserlichtbündels ausgeblendet bleiben.

Mittels des axialen Kappenteils 8 ist die Applikationskappe 2 im Arbeitskanal 6 des Endoskops geführt, wobei die Gesamtlänge von Aufnahmehülse 14 und Kappenabschnitt 24 dem maximalen Verschiebeweg der Applikationskappe 2 entspricht, so daß vom proximalen Ende der Lichtleitfaser 4 über die Applikationskappe 2 Druck auf das Gewebe ausgeübt werden kann, ohne daß die Lichtleitfaser 4 unabgestützt aus dem distalen Ende des Arbeitskanals 6 ausgeschoben wird.

Zusätzlich zur Führungsfunktion übernimmt die Aufnahmehülse 14 auch die Aufgabe einer thermischen Isolation, durch die die temperaturempfindlichen Teile, also bei dem gezeigten Ausführungsbeispiel die Verklebung 20, vor einer übermäßigen Wärmeeinwirkung seitens des sich bei der Gewebevaporisation stark erhitzenden Biegeteils 10 geschützt werden.

Bei einem typischen Ausführungsbeispiel besitzt die Applikationskappe 2 eine Gesamtlänge von 25 mm, die Aufnahmehülse 14 eine Länge von 18mm, der Kappenabschnitt 24 ist 2 bis 4 mm und der Biegeteil 10 ist zwischen 3 und 5 mm lang; der Kappen-Außendurchmesser beträgt etwa 1,6 mm und der Abstand des radial äußersten Flächenpunktes P von der Mittelachse des Kappenteils 8 liegt bei 1,5 mm. Der Divergenzwinkel α des Therapielichts im Kappenmaterial hat typischerweise einen Wert zwischen 3° und 10°, und das Biegeteil 10 ist relativ zum Kappenteil 8 um einen Winkel β von 10° bis 20° abgebogen.

## Patentansprüche

1. Laserinstrument zur Kontaktvaporisation von flüssigkeitshaltigem Gewebe, mit einer optisch transparenten, mit dem distalen Ende (22) eines Laserlichtleiters verkoppelten Applikationskappe (8), bestehend aus einem das Lichtleiterende (22) axial aufnehmenden Kappenteil (8) und einem von diesem abgebogenen, die Applikationsspitze bildenden Biegeteil (10),
**dadurch gekennzeichnet, daß**
der axiale Kappenteil (8) in Strahlrichtung soweit über das distale Lichtleiterende (22) verlängert ist, daß der biegeäußere Randstrahl (Ra) eines aus dem Lichtleiterende austretenden Strahlenbündels im Übergangsbereich zwischen axialem Kappenteil und Biegeteil (10) austritt, und daß der Biegewinkel (β) des Biegeteils mindestens ebenso groß wie der Divergenzwinkel (α) des Strahlenbündels in der Kappe (2) und unter Berücksichtigung der Länge des Biegeteils derart bemessen ist, daß der biegeinnere Randstrahl (Ri) im Bereich zwischen dem radial äußersten Flächenpunkt (P) und der äußersten distalen Spitze (S) des Biegeteils austritt.

2. Laserinstrument nach Anspruch 1,
**dadurch gekennzeichnet, daß**
zumindest der Bereich des Biegeteils (10), aus welchem das Strahlenbündel austritt, eine angerauhte Oberfläche aufweist.

3. Laserinstrument nach Anspruch 1 oder 2,
**dadurch gekennzeichnet, daß**
der axiale Kappenteil (8) proximalseitig als den Faserkern (12) des Lichtleiters (4) mit radialem Abstand umschließende Aufnahmehülse (14) ausgebildet ist.

4. Laserinstrument nach Anspruch 3,
**dadurch gekennzeichnet, daß**
der Lichtleiter (4) mit dem proximalen Ende der Aufnahmehülse (14) verklebt und diese entsprechend der Hülsenlänge als thermische Isolationszone für die Klebestelle (20) ausgebildet ist.

5. Laserinstrument nach einem der vorhergehenden Ansprüche,
**dadurch gekennzeichnet, daß**
die Applikationskappe (2) im Arbeitskanal (6) eines Endoskops verschieblich geführt ist.

6. Laserinstrument nach Anspruch 5 in Verbindung mit Anspruch 3 oder 4,
**dadurch gekennzeichnet, daß**
die Applikationskappe (2) mittels der Aufnahmehülse (14) im Arbeitskanal (6) des Endoskops geführt ist.

7. Laserinstrument nach einem der vorhergehenden Ansprüche,
**dadurch gekennzeichnet, daß**
der Lichtleiter (4) am distalen Lichtleiterende (22) mit dem axialen Kappenteil (8) verschmolzen ist.

8. Laserinstrument nach einem der vorhergehenden Ansprüche,
**dadurch gekennzeichnet, daß**
die Applikationskappe (2) und der Faserkern (12) des Lichtleiters (4) aus Materialien mit im wesentlichen gleichem Brechungsindex bestehen.

9. Laserinstrument nach Anspruch 8,
**dadurch gekennzeichnet, daß**
die Applikationskappe (2) und der Faserkern (12) des Lichtleiters (4) aus Quarzglas bestehen.

10. Laserinstrument nach einem der vorhergehenden Ansprüche,
**dadurch gekennzeichnet, daß**
die Mittelachsen des Biegeteils (10) und des axialen Kappenteils (8) relativ zueinander unter einem Biegewinkel (β) zwischen 5° und 30° abknickend verlaufen.

## Claims

1. Laser instrument for vaporization by contact of tissue containing liquid, having an optically transparent application cap coupled to the distal end of a laser light guide, consisting of a cap part which receives the light guide end axially, and a bent part bending from the latter and forming the application tip,
**characterized in that**
the axial cap part (8) extends beyond the distal light guide end (22) in the direction of the ray to an extent such that the outer bend marginal ray (Ra) of the ray bundle, which emerges from the light guide end, emerges in the transition region between the axial cap part and the bent part (10), and that the bending angle (β) of the bent part is at least equally as great as the angle of divergence (α) of the ray bundle in the cap (2), and, taking into account the length of the bent part, is dimensioned in such a way that the inner bend marginal ray (Ri) emerges in the region between the radially outermost surface point (P) and the outermost distal tip (S) of the bent part.

2. Laser instrument according to claim 1, **characterized in that** at least the region of the bent part (10) exhibits a roughened surface from which the ray bundle emerges.

3. Laser instrument according to claim 1 or 2, **characterized in that** the axial cap part (8) is configured at the proximal end as a receiving sleeve (14), which encloses the fiber core (12) of the light guide (4) at a radial distance.

4. Laser instrument according to claim 3, **characterized in that** the light guide (4) is bonded to the proximal end of the receiving sleeve (14), and the latter is designed according to the length of the sleeve as a thermal insulation zone for the bonding surface (20).

5. Laser instrument according to one of the preceding claims, **characterized in that** the application cap (2) is guided in sliding fashion within the working channel (6) of an endoscope.

6. Laser instrument according to claim 5 in combination with claim 3 or 4, **characterized in that** the application cap (2) is guided by means of the receiving sleeve (14) within the working channel (6) of the endoscope.

7. Laser instrument according to one of the preceding claims, **characterized in that** the light guide (4) at the distal light guide end (22) is fused with the axial cap part (8).

8. Laser instrument according to one of the preceding claims, **characterized in that** the application cap (2) and the fiber core (12) of the light guide (4) are made of materials with essentially the same index of refraction.

9. Laser instrument according to claim 8, **characterized in that** the application cap (2) and the fiber core (12) of the light guide (4) are made of quartz glass.

10. Laser instrument according to one of the preceding claims, **characterized in that** the center lines of the bent part (10) and the axial cap part (8) progress at a bending angle (β) of between 5° and 30° relative to each other.

## Revendications

1. Instrument laser pour la vaporisation par contact de tissu contenant du liquide, ayant un capuchon d'application (8) optiquement transparent couplé à l'extrémité (22) distale d'un guide de lumière laser, comportant une partie axiale de capuchon (8) recevant l'extrémité (22) du conducteur de lumière et une partie pliée (10) courbée par rapport à celle-ci et formant la pointe d'application,
**caractérisé en ce que**
la partie axiale de capuchon (8) est prolongée dans la direction du faisceau au-delà de l'extrémité distale (22) du guide de lumière, **en ce que** le rayon de bord dévié (Ra) extérieurement d'un faisceau de rayons sortant de l'extrémité du guide de lumière sort dans une zone de transition entre la partie axiale de capuchon et la partie pliée (10) et **en ce que** l'angle de pliage ( β) de la partie pliée est au moins aussi grand que l'angle de divergence (α) du faisceau de rayons dans le capuchon (2) et est dimensionnée en fonction de la longueur de la partie pliée de telle sorte que le rayon de bord (Ri) dévié intérieurement sorte dans la zone entre le point (P) de surface radialement le plus extérieur et la pointe (S) distale la plus extérieure de la partie pliée.

2. Instrument laser selon la revendication 1,
**caractérisé en ce que**
au moins la zone de la partie pliée (10), dont sort le faisceau de rayons, présente une surface rendue rugueuse.

3. Instrument laser selon la revendication 1 ou 2,
**caractérisé en ce que**
la partie axiale de capuchon (8) est conçue côté proximal comme une douille de logement (14) entourant à distance radiale le noyau de fibre (12) du guide de lumière (4).

4. Instrument laser selon la revendication 3,
**caractérisé en ce que**
le guide de lumière (4) est collé à l'extrémité proximale de la douille de logement (14) et celle-ci est réalisée en fonction de la longueur de douille comme zone d'isolation thermique pour le point de collage (20).

5. Instrument laser selon l'une quelconque des revendications précédentes,
**caractérisé en ce que**
le capuchon d'application (2) est guidé de façon coulissante dans le conduit de travail (6) d'un endoscope.

6. Instrument laser selon la revendication 5 en liaison avec la revendication 3 ou 4,
**caractérisé en ce que**
le capuchon d'application (2) est guidé au moyen de la douille de logement (14) dans le conduit de travail (6) de l'endoscope.

7. Instrument laser selon l'une quelconque des
revendications précédentes,
**caractérisé en ce que**
le guide de lumière (4) est fondu sur l'extrémité (22) distale du guide de lumière avec la partie axiale de capuchon (8).

8. Instrument laser selon l'une quelconque des revendications précédentes,
**caractérisé en ce que**
le capuchon d'application (2) et le noyau de fibre (12) du guide de lumière (4) se composent de matériaux présentant un indice de réfraction sensiblement identique.

9. Instrument laser selon la revendication 8,
**caractérisé en ce que**
le capuchon d'application (2) et le noyau de fibre (12) du guide de lumière (4) sont en verre de quartz.

10. Instrument laser selon l'une quelconque des
revendications précédentes,
**caractérisé en ce que**
les axes médians de la partie pliée (10) et de la partie axiale de capuchon (8) sont agencés l'un par rapport à l'autre en formant une pliure selon un angle de pliage (β) compris entre 5°et 30°.
